# EUROPEAN PATENT SPECIFICATION

(11) **EP 0 178 106 B1**
(45) Date of publication and mention of the grant of the patent: **31.03.1993**
(21) Application number: 85306971.4
(22) Date of filing: 30.09.1985
(51) Int. Cl.: C12P 13/00, C12P 13/02, C08G 69/14, C12N 9/78

(54) **Enzymatic process**
Enzymatisches Verfahren
Procédé enzymatique

(30) Priority: 01.10.1984 DK 4695/84; 12.04.1985 DK 1681/85
(43) Date of publication of application: 16.04.1986
(73) Proprietor: NOVO NORDISK A/S, 2880 Bagsvaerd (DK)
(72) Inventor: Godtfredsen, Sven Erik, DK-3500, Vaerloese (DK); Andresen, Otto, DK-3660 Stenloese (DK)
(74) Representative: Brown, John David

(56) References cited:
- US-A- 4 629 700
- CHEMICAL ABSTRACTS, vol. 94, no. 13, 30th March 1981, page 406, abstract no. 99462c, Columbus, Ohio, US; M. KUWAHARA et al.: "Metabolism of succinonitrile in Aeromonas sp", & HAKKO KOGAKU KAISHI 1980, 58(6), 441-7
- M. WINDHOLZ et al.: "The Merck Index", tenth edition, 1983, page 6580, Merck & CO., Rahway, N.J. US
- PATENT ABSTRACTS OF JAPAN, vol. 6, no. 110 (C-109)[988], 22nd June 1982; & JP-A-57 39 792 (NITTO KAGAKU KOGYO K.K.) 05-03-1982
- AGRICULTURAL AND BIOLOGICAL CHEMISTRY, vol. 46, no. 5, May 1982, pages 1183-1189, Tokyo, JP; Y. ASANO et al.: "A new enzymatic method of acrylamide production"

## Description

The present invention relates to a process for transforming selectively only or mainly one of the cyano groups in dinitriles into the corresponding cyano carboxylic acid, cyano carboxylic acid amides, cyano carboxylic acid ester or cyano carboxylic acid thio ester with the aid of enzymes hereinafter designated mononitrilases.

### BACKGROUND OF THE INVENTION

The occurrence of nitrile converting enzymes is well established. Among them, those nitrile converting enzymes which catalyze the transformation of nitriles into the corresponding carboxylic acids have been particularly well studied by workers in the art. Investigations carried out, using such enzymes, indicate that the transformation of nitriles by nitrile converting enzymes may proceed at the molecular level by various mechanisms. Scheme 1 below summarizes one hypothetical scheme which has been forwarded to explain transformation of nitriles into carboxylic acids. 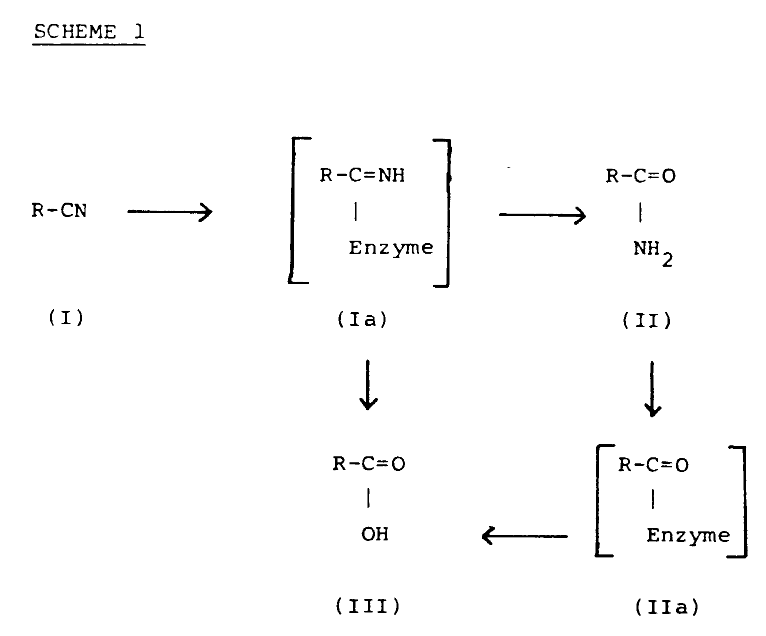
wherein R indicates a radical, i.e., the balance of the nitrile, and "Enzyme" indicates a nitrile converting enzyme.

As indicated in Scheme 1, it is speculated that nitriles of the general Formula I attacked by the enzyme goes to the transient species of the general Formula Ia which, in the presence of water, breaks down to a carboxylic acid of the general Formula III or to the corresponding carboxylic acid amide of the general Formula II. The amide of Formula II may subsequently be converted into the transient species of the Formula IIa and, ultimately, into the corresponding carboxylic acid of Formula III. The nitrile converting enzymes catalyzing the transformations from a compound of Formula I into a compound of Formula II, from a compound of Formula II into a compound of Formula III and from a compound of Formula I into a compound of Formula III are denoted nitrile hydratases, amidases and nitrilases, respectively. The term nitrile converting enzyme used herein thus comprises nitrilases and nitrile hydratases optionally containing amidases.

The range of nitriles amenable for enzyme catalyzed transformation is very wide indeed. As pointed out in a recent review on bioconversion of nitriles by Jallageas et al., Adv.Biochem.Engineer., 14 (1980), 1 et seq., it seems that any nitrile may undergo enzymatic conversion. Also, this review describes microbial sources of nitrile converting enzymes with an exceedingly broad specificity naming species of Bacillus, Bacteridium, Micrococcus and Brevibacterium. The authors believe their microbial nitrilases to be non-specific. It would be expected also that microbial nitrilases will attack both cyano groups in dinitriles.

A similar description appears in U.S. patent specification No. 3,940,316 by the Jallageas research group, namely a process by which nitriles are hydrolysed into acids using bacteria showing nitrilasic activity. The hydrolysis are stated to be complete, i.e. dinitriles are completely hydrolysed.

A further analogous description by the same research group appears in U.S. patent specification No. 4,001,081 according to which two specific dinitriles are converted into the corresponding diamides using bacteria having nitrilasic activity, i.e., both cyano groups are converted, vide Examples 5 and 11 therein.
*
Surprisingly, it has now been found that certain nitrile converting enzymes denoted mononitrilases, among them even those possessing a broad substrate specificity, may be utilized for selective transformation of only or mainly one of the cyano groups in dinitriles. In other words, it has been found that certain nitrile converting enzymes are, to all practical purposes, mononitrilases and this discovery makes possible practice of this invention.
* Kuwahara et al., Hakkokagaku Kaishi, 58, (1980) 441-4477 discloses the conversion of succinonitrile to succinic acid in Aeromonas using inter alia a nitrilase.

The present invention comprises a process for converting a dinitrile into a corresponding cyano carboxylic acid, cyano carboxylic acid amide, cyano carboxylic acid ester or cyano carboxylic acid thio ester which comprises treating the dinitile with a mononitrilase generated from a microorganism of the genus Bacteridium, Bacillus, Brevibacterium, Rhodococcus or Micrococcus and, thereafter, if desired, recovering the desired product.

For example, the following microbial species may be utilized for generation of mononitrilases: Species of Micrococcus, Bacillus, Brevibacterium, Bacteridium, strain A4 (deposited at Laboratory of Microbiology (hereinafter designated LMD), the Netherlands, under No. LMD 79.2, and deposited at the National Collection of Industrial Bacteria (hereinafter designated NCIB) on 12 April 1985 under No. 12070), strains N-771, N-774 and N-775 (deposited at Fermentation Research Institute (hereinafter designated FRI), Japan, under No. 4445, 4446 and 4447, respectively) and strains R 332 (deposited at Centraalbureau voor Schimmelcultures (hereinafter designated CBS), the Netherlands), R 340 (CBS No. 495.74), R 341 (CBS No. 496.74), A 111 (CBS No. 497.74), B 222 (CBS No. 498.74), A 112, A 13, A 141, A 142, B 211, B 212, B 221, C 211 (CBS No. 499.74), R 21, R 22, R 311, R 312 (CBS No. 717.73) and R 331 described in U.S. patent specificaton No. 4,001,081. On 12 April 1985, strains deposited under Nos. CBS 496.74, 494.74, 495.74, 717.73, 498.74 and 497.74, respectively, were re-deposited under Nos. NCIB 12067 through 12069 and 12071 through 12073, respectively.

The suitability of a specific nitrile converting enzyme for use in the process of this invention can be tested by monitoring the enzyme catalyzed conversion of the dinitrile to be converted by high pressure liquid chromatography (hereinafter HPLC), gas liquid chromatography, nuclear magnetic resonance (hereinafter designated NMR) spectroscopy or other analytical techniques, for example, detection of ammonia liberated during the reaction. Nitrile converting enzymes which attack only one of the cyano groups in a specific dinitrile are mononitrilases. Also, nitrile converting enzymes which, within a certain period of time, attack only one of the cyano groups in a specific dinitrile are suited to practice of this invention. Furthermore, nitrile converting enzymes which preferentially hydrolyse one of the cyano groups in a specific dinitrile and which, within a certain period of time, attack also the second cyano group to a minor degree, for example, less than 25%, preferably less than 5%, by the time the first cyano group has been transformed may also be used in practice of this invention (although in such instances, the purification of the desired compound (formula V) becomes more difficult). Such nitrile converting enzymes are mononitrilases as a practical matter and are herein considered to be mononitrilases for the reason that they have the mononitrilase specificity needed for the process of this invention. According to the above explanation, mononitrilases encompass mononitrile hydratases optionally containing amidases.

The mononitrilase preparations used in the process of this invention comprises any preparation which is able to convert only or mainly one of the cyano groups present in a dinitrile into a carboxylic acid group, into a carboxylic acid amide group, into a carboxylic acid ester group or into a carboxylic acid thio ester group. The mononitrilase preparations may be highly purified mononitrilases, crude enzymes isolated from microorganisms, ruptured cells of said microorganisms or intact cells. It may be advantageous to utilize the mononitrilases in an immobilized form which can readily be prepared according to known methods. The mononitrilases may also be used in a chemically modified form exhibiting greater stability and activity than the native enzymes.

Nitrile converting enzymes exhibit broad specificity. Also, mononitrilases are broadly applicable to the desired conversion of dinitriles according to this invention.

More specifically, cyano carboxylic acids, cyano carboxylic acid amides, cyano carboxylic acid esters and cyano carboxylic acid thio esters of the general Formula V

NC-X¹-X²-COR¹ (V)

wherein R¹ represents -NR²R³, -OR² or -SR², wherein R² and R³ are the same or different, each representing hydrogen, lower alkyl, aryl or aryl(lower alkyl), and X¹ and X² are the same or different each representing straight or branched alkylene or arylene each of which may be substituted with one, two or more of the following groups: hydroxy, amino, carboxy, nitro, cyano, aryl, lower alkoxy, lower alkylthio or lower alkylene, with the proviso that, furthermore, X² may be a valence bond, can be prepared by converting only or mainly one of the cyano groups in the corresponding dinitrile of the general Formula IV

NC-X¹-X²-CN (IV)

wherein X¹ and X² each are as defined above, with a mononitrilase.

The term "lower" used in connection with some of the substituents or a part of a substituent in the compounds of formula IV and V indicates that, preferably, the group in question contains not more than six carbon atoms, preferably not more than four carbon atoms. Hence, lower alkyl is, preferably, methyl, ethyl and propyl. Aryl is, preferably, phenyl or naphthyl. Aryl(lower alkyl) is, preferably, benzyl. Lower alkylene is, preferably, methylene, ethylene, propylene and 1,4-butylene. Lower alkoxy is, preferably, methoxy and ethoxy. Lower alkylthio is, preferably, methylthio or ethylthio. Arylene is, preferably, o-, m- and p-phenylene. Preferably, the alkylene residue designated X¹ or X² contains not more than 22 carbon atoms, preferably not more than 18 carbon atoms.

Specific and preferred examples of dinitriles of Formula IV are dinitriles of malonic acid which may be substituted with 1,5-pentylene or ethylene, of succinic acid, of glutaric acid and of adipic acid.

The process of this invention can be carried out by treating dinitriles (Formula IV) in solution, for example, an aqueous solution, with a mononitrilase. The concentration of the dinitrile (Formula IV) in the reaction medium may be varied, as required, from dilute to highly concentrated solutions. Organic solvents may be added to the reaction mixture or the enzymatic reaction may be carried out in an essentially water-free environment consisting, for example, of the pure starting material (Formula IV) or a mixture of starting material (Formula IV) and an organic solvent. The organic solvent may, inter alia, be dioxane, dimethylsulfoxide or N,N-dimethylformamide, which may be mixed with water, for example, 30/70 (volume/volume). The transient species (Formula Ia and IIa) may by attack of nucleophiles other than water such as, for example, alcohols, thiols and amines, provide the corresponding carboxylic acid intermediates, for example, esters, thio esters and amides. By carrying out the process of this invention in the presence of alcohols, thioles or amines, carboxylic acid amides, carboxylic acid ester or carboxylic acid thio esters may thus be synthezised.

Conveniently, the enzymatic conversion is followed by analyzing aliquots of the reaction mixture, for example, with HPLC, gas liquid chromatography, NMR spectroscopy or, when amidases or nitrilases are used, determination of ammonia. The reaction is stopped at the desired time, for example, by removal of the enzyme, by heating the reaction mixture or by acidification, for example, to a pH value of about 1, for example, with hydrochloric acid. The reaction time thus determined may be from less than an hour to several hours and it may be desirable to adjust the pH value of the reaction mixture and stir the reaction mixture. Thereafter, the desired compound (Formula V) is isolated in a manner known per se, optionally after removal of the mononitrilase in a manner known per se, for example, by filtration or centrifugation.

A convenient reaction temperature is in the range from about 0 to 50°C, preferably from about 30 to 40°C. Conveniently, the pH value of the reaction medium is in the range from about 4.5 to 10.5, preferably from about 6 to 9.

The yield obtained depends, inter alia, on the concentration of enzyme and substrate used, on the temperature and the pH value of the reaction medium and the reaction time.

A preferred utility of this invention concerns the preparation of epsilon-caprolactam, which is used for preparation of nylon 6. By the process of this invention, adipic acid dinitrile can be converted into adipic acid mononitrile. The latter compound may be hydrogenated by known-to-the-art methods into 6-amino-n-caproic acid, which subsequently may be dehydrated into epsilon-caprolactam by known-to-the-art methods. Alternatively, adipic acid dinitrile is, by the process of this invention, converted into the corresponding cyano carboxylic acid amide, ester or thio ester, which may be hydrogenated and subsequently cyclized into epsilon-caprolactam. Quite similarly, other lactams may be generated from the corresponding dinitriles.

A further preferred utility of this invention concerns the preparation of chirale compounds. Prochirale substrates of the general Formula IV may thus, by the process of this invention, be transformed into chirale products of the general Formula V. For example, prochirale dialkylated derivates of malonic acid dinitrile or related aliphatic dinitriles may, by the process of this invention, be converted into the corresponding chirale monocyano compounds.

The process of this invention is further illustrated by the following examples which, however, are not construed as limiting. The examples illustrate some preferred embodiments of the process of this invention. The letters TM after a name indicate that it is a Trademark.

### Example 1

500 ml of a medium containing Peptone (6 g/l), Pepticase (4 g/l), beef extract (1.5 g/l), yeast extract (3 g/l) and glucose (10 g/l) in conically shaped shake flasks was inoculated with 10 ml of a 24 hours old culture of strain B222 (the CBS No. 498.74, referenced in U.S. patent specification No. 4,001,081, there this organism is designated Brevibacterium, however, it has also been classified as Rhodococcus). The cultures were incubated for 24 hours at 37°C by orbital shaking. The generated biomass was isolated by centrifugation, washed three times with 100 ml portions of 0.1 M phosphate buffer at pH 7.0, and finally suspended in 10 ml of said buffer. The enzymatically active cell mass suspension was added to 15 ml of 800 mM adipic acid dinitrile in 0.1 M phosphate buffer at pH 7. The resulting reaction mixture was stirred at 22°C for three hours, extracted with three volumes of diethyl ether, acidified by addition of 1 M phosphoric acid and extracted with three volumes of diethyl ether. The combined ether phases were washed with saline, dried over sodium sulphate and concentrated in vacuo to provide the cyanocarboxylic acid in 90% yield. The product was homogeneous as judged by thin layer chromatography on silica gel plates and exhibited an IR-spectrum identical to that of an authentic specimen of the cyanocarboxylic acid.

### Example 2

A 30 mM solution of glutaric acid dinitrile in 0.1 M phosphate buffer at pH 7 was treated substantially as described in Example 1 with the mononitrilase cell mass product. The liberation of ammonia in the reaction mixture was monitored and when conversion into the monocyanomonocarboxylic acid was judged to be complete, the enzymatic reaction was stopped by lowering pH of the reaction mixture to 1 by addition of 1 M hydrochloric acid. Insoluble material was removed from the reaction mixture by centrifugation whereupon the reaction products were removed from the mixture by contineous extraction with diethyl ether. The etheral solution of the reaction product was finally dried over sodium sulfate and concentrated in vacuo to provide the desired monocyanomonocarboxylic acid in 75% yield.

### Example 3

A 30 mM solution of malonic acid dinitrile in 0.1 M phosphate buffer (pH: 7) was treated with the mononitrilase analogously as described in Example 2. The reaction product was isolated by repeated extraction of the reaction mixture with chloroform after acidification and saturation with sodium chloride. Subsequent evaporation of the applied chloroform served to provide the desired monocyanomonocarboxylic acid in 60% yield.

### Example 4

A 10 mM solution of sebacic acid dinitrile in 0.1 M phosphate buffer at pH 7 containing 20% dioxane was treated with the mononitrilase analogously as described in Example 1. The reaction product isolated as described in Example 2 was shown to be the desired monocyanomonocarboxylic acid by comparison with an authentic specimen of this compound. A yield of 70% was achieved.

### Example 5

1,4-dicyanobenzene (10 mM) in a phosphate buffer (0.1 M, pH 7) containing 20% dioxane was treated with the mononitrilase analogously as described in Example 1. The progress of the enzyme catalyzed conversion was monitored by analyzing, using HPLC, the reaction mixture at 15 minutes intervals. The reaction was stopped by acidification with 1 M hydrochloric acid when conversion to the desired monocyanomonocarboxylic acid was judged to be optimal whereupon work-up analogously as described in Example 1 furnished the desired product in 65% yield.

### Example 6

3,3-dimethylglutaric acid dinitrile was treated with the mononitrilase analogous to the procedure described in Example 1 to provide 3,3-dimethyl-4-cyanobutyric acid in 85% yield.

### Example 7

3-methyl-3-hydroxyglutaric acid dinitrile was treated with the mononitrilase analogously as described in Example 2 to provide the desired 3-methyl-3-hydroxy-4-cyanobutyric acid in 65% yield.

### Example 8

3-methyl-3-aminoglutaric acid dinitrile was converted in manner analogous to that described in Example 7 to provide 3-ethyl-3-amino-4-cyanobutyric acid in 55% yield.

### Example 9

a) Strain B222 was cultivated in the medium described in Example 1 supplemented with 100 ppm of monofluoroacetamide. After 14 days, the generated biomass was isolated substantially as described in Example 1 and, subsequently, added to 1000 ml of a 0.4 M solution of adiponitrile in 10 mM ammonium bicarbonate buffer at pH 7.5. Aliquots of the reaction mixture were collected at 5 minutes intervals and analyzed by HPLC using a reverse phase Suphelco™ C-18 column and an eluent consisting of 10 mM phosphate buffer at pH 7. After 25 minutes the reaction mixture was found to contain the monoamide derivative of adiponitrile in a quantity corresponding to a yield of 99.5%. The mononitrilase was immediately removed from the reaction mixture by filtration whereupon the resulting solution was freeze-dried to provide the desired 5-cyanopentanoic acid amide in 98% yield. The compound was homogeneous as judged by HPLC and thin layer chromatography and, in these tests, behaved in a way identical to an authentic specimen of the desired compound.
b) Reacting adiponitrile with the Brevibacterium enzyme prepared as described above, with the proviso that the reaction time was 3 hours instead of 25 minutes, 5-cyanopentanoic acid amide was still obtained.

### Example 10

Glutaric acid dinitrile was treated in manner analogous to that described in Example 9 to provide the corresponding monoamidomonocyano compound in 95% yield.

### Example 11

Sebacic acid dinitrile was converted to the corresponding monoamidomonocyano derivative in manner analogous to that described in Example 9. A yield of 75% was achieved.

### Example 12

3-methyl-3-hydroxyglutaric acid dinitrile was treated with the mononitrilase analogously as described in Example 9 to provide 3-methyl-3-hydroxy-4-cyanoglutaric acid amide in 80% yield.

### Example 13

A mononitrilase preparation generated as described in Example 9 was suspended in a 3% aqueous solution of sodium alginate. The resulting suspension was transferred dropwise to an aqueous 0.1 M calcium chloride solution. The resulting pellets were stirred in the calcium chloride solution for 3 hours and, subsequently, washed with several portions of water containing 0.9% of sodium chloride. The immobilized mononitrilase was then packed into a column and an aqueous solution containing adiponitrile (0.4 M) and ammonium bicarbonate (5 mM, pH 7.5) was passed through the resulting enzyme reactor. Samples of the eluent from the reactor were analyzed by HPLC by the method used in Example 9 and the flow through the reactor was adjusted to secure a yield of the desired monoamidomonocyano compound of 95%.

### Example 14

A nitrilase preparation was made available from NCIB 12067 analogously as described in Example 1. The resulting enzymatically active cells were suspended in a 30 mM solution of adipodinitrile in 10 mM phosphate buffer which was stirred efficiently at room temperature. The progress of the hydrolytic reaction was monitored by HPLC which indicated a yield of the monoamido compound in 97% yield.

### Example 15

An enzyme preparation prepared as described in Example 14 was suspended in 10 mM phosphate buffer to which pure adipodinitrile was added until a two-phase system of nitrile and water phase was achieved. The mixture was then efficiently stirred in order to emulsify the dinitrile in the aqueous enzyme containing phase. Aliquots of the reaction mixture were analyzed as indicated in Example 9. A 76% yield of the desired monoamide was achieved after work up in the usual manner.

### Example 16

Using an enzyme preparation furnished as described in Example 14, malonic acid dinitrile was converted into the corresponding monocyanomonocarboxylic acid in a manner analogous to that described in Example 3.

### Example 17

Using an enzyme preparation as described in Example 14 and the procedure outlined in Example 6, 3,3-dimethyl-4-cyanobutyric acid was prepared in 70% yield.

## Claims

1. A process for converting a dinitrile into the corresponding cyano carboxylic acid, cyano carboxylic acid amide, cyano carboxylic acid ester or cyano carboxylic acid thio ester which comprises treating the dinitrile with a mononitrilase generated from a microorganism of the genus Bacteridium, Bacillus, Brevibacterium, Rhodococcus or Micrococcus and, thereafter, if desired, recovering the desired product.

2. A process according to Claim 1 wherein the cyano carboxylic acids, cyano carboxylic acid amides, cyano carboxylic acid esters or cyano carboxylic acid thio esters have the general formula V
NC-X¹-X²-COR¹ (V)
wherein R¹ represents -NR²R³, -OR² or -SR², wherein R² and R³ are the same or different, each representing hydrogen, lower alkyl, aryl or aryl (lower alkyl), and X¹ and X² are the same or different each representing straight or branched alkylene or arylene each of which may be substituted with one, two or more of the following groups: hydroxy, amino, carboxy, nitro, cyano, aryl, lower alkoxy, lower alkylthio or lower alkylene, with the proviso that, furthermore, X² may be a valence bond.

3. A process, according to Claim 1 or 2, characterized in that the conversion is interrupted before the cyano group of the desired product (formula V) has, in a substantial degree, been transformed by the mononitrilase.

4. A process, according to any one of the preceding Claims, characterized in that less than 25%, preferably less than 5%, of the desired product (formula V) has been transformed.

5. A process, according to any one of the preceding Claims, characterized in that the dinitrile (formula IV) is prochirale and the desired product (formula V) is chirale.

6. A process, according to any one of the preceding Claims, wherein the mononitrilase is an immobilized enyzme.

7. A process, according to any one of the preceding Claims, characterized in that the mononitrilase exhibits enzyme chemical and immunological properties essentially identical to those of the mononitrilase derived from the strains deposited under Nos. NCIB 12067 through 12073.

8. A process, according to any one of the preceding Claims, wherein the dinitrile is selected from the group consisting of malonic acid dinitrile, glutaric acid dinitrile, adipic acid dinitrile, sebacic acid dinitrile, 3,3-dimethyl-glutaric acid dinitrile, 3-methyl-3-hydroxyglutaric acid dinitrile, 3-ethyl-3-aminoglutaric acid dinitrile and 1,4-dicyanobenzone.

9. A process, for preparing Nylon 6, which process comprises preparing a compound of the general formula Va
NC-(CH₂)₄-COR¹ (Va)
wherein R¹ is as defined in Claim 2 by the process according to any one of the preceding Claims.

10. A process, according to Claim 8, wherein the dinitrile is adipic acid dinitrile.

## Patentansprüche

1. Verfahren zur Umwandlung eines Dinitrils in die entsprechende Cyanocarbonsäure, das entsprechende Cyanocarbonsäureamid, den entsprechenden Cyanocarbonsäureester oder den entsprechenden Cyanocarbonsäurethioester, welches umfaßt, daß das Dinitril mit einer Mononitrilase behandelt wird, die aus einem Mikroorganismus der Gattung Bacteridium, Bacillus, Brevibacterium, Rhodococcus oder Mikrococcus gewonnen ist, und danach, falls erwünscht, das gewünschte Produkt gewonnen wird.

2. Verfahren nach Anspruch 1, dadurch gekennzeichnet, daß die Cyanocarbonsäuren, Cyanocarbonsäureamide, Cyanocarbonsäureester oder Cyanocarbonsäurethioester die allgemeine Formel V besitzen
NC-X¹-X²-COR¹ (V),
in der R¹ -NR²R³, -OR² oder -SR² darstellt, wobei R² und R³ identisch oder verschieden sind und jeweils Wasserstoff, Niederalkyl, Aryl oder Aryl(Niederalkyl) darstellen, und X¹ und X² identisch oder verschieden sind und jeweils geradkettiges oder verzweigtes Alkylen oder Arylen darstellen, von denen jedes mit einer, zwei oder mehreren der folgenden Gruppen substituiert sein kann: Hydroxy, Amino, Carboxy, Nitro, Cyano, Aryl, Niederalkoxy, Niederalkylthio oder Niederalkylen, mit der Maßgabe daß außerdem X² eine Valenzbindung sein kann.

3. Verfahren nach Anspruch 1 oder 2, dadurch gekennzeichnet, daß die Umwandlung unterbrochen wird, bevor die Cyanogruppe des gewünschten Produktes (Formel V) in einem wesentlichen Umfang von der Mononitrilase umgewandelt worden ist.

4. Verfahren nach einem der vorangehenden Ansprüche, dadurch gekennzeichnet, daß weniger als 25 %, vorzugsweise weniger als 5 %, des gewünschten Produktes (Formel V) umgewandelt worden sind.

5. Verfahren nach einem der vorangehenden Ansprüche, dadurch gekennzeichnet, daß das Dinitril (Formel IV) prochiral ist und das gewünschte Produkt (Formel V) chiral ist.

6. Verfahren nach einem der vorangehenden Ansprüche, dadurch gekennzeichnet, daß die Mononitrilase ein immobilisiertes Enzym ist.

7. Verfahren nach einem der vorangehenden Ansprüche, dadurch gekennzeichnet, daß die Mononitrilase enzymchemische und immunologische Eigenschaften zeigt, die im wesentlichen identisch sind mit denjenigen der Mononitrilase, die aus den Stämmen gewonnen ist, die unter den Nummern NCIB 12067 bis 12073 hinterlegt worden sind.

8. Verfahren nach einem der vorangehenden Ansprüche, dadurch gekennzeichnet, daß das Dinitril ausgewählt ist aus der Gruppe, die aus Malonsäuredinitril, Glutarsäuredinitril, Adipinsäuredinitril, Sebacinsäuredinitril, 3,3-Dimethylglutarsäuredinitril, 3-Methyl-3-hydroxyglutarsäuredinitril, 3-Ethyl-3-aminoglutarsäuredinitril und 1,4-Dicyanobenzol besteht.

9. Verfahren zur Herstellung von Nylon 6, wobei das Verfahren die Herstellung einer Verbindung der allgemeinen Formel Va
NC-(CH₂)₄-COR¹ (Va),
in der R¹ wie in Anspruch 2 definiert ist, mit dem Verfahren gemäß einem der vorangehenden Ansprüche umfaßt.

10. Verfahren nach Anspruch 8, dadurch gekennzeichnet, daß das Dinitril Adipinsäuredinitril ist.

## Revendications

1. Procédé pour transformer un dinitrile en l'acide cyanocarboxylique, l'amide d'acide cyanocarboxylique, l'ester d'acide cyanocarboxylique ou le thioester d'acide cyanocarboxylique correspondants, qui comprend le traitement du dinitrile avec une mononitrilase produite par un microorganisme du genre Bacteridium, Bacillus, Brevibacterium, Rhodococcus ou Micrococcus puis, si désiré, récupération du produit désiré.

2. Procédé selon la revendication 1 dans lequel les acides cyanocarboxyliques, les amides d'acides cyanocarboxyliques, les esters d'acides cyanocarboxyliques ou les thioesters d'acides cyanocarboxyliques ont la formule générale V
NC-X¹-X²-COR¹ (V)
dans laquelle R¹ représente -NR²R³, -OR² ou -SR², où R² et R³ sont identiques ou différents et représentent chacun l'hydrogène, un alkyle inférieur, un aryle ou un aryl(alkyle inférieur), et X¹ et X² sont identiques ou différents et représentent chacun un alkylène linéaire ou ramifié ou un arylène, chacun d'eux pouvant être substitué par un, deux ou plusieurs des groupes suivants: hydroxy, amino, carboxy, nitro, cyano, aryle, alcoxy inférieur, alkylthio inférieur ou alkylène inférieur, sous réserve que X² puisse être en outre une liaison de valence.

3. Procédé selon la revendication 1 ou 2, caractérisé en ce que la transformation est arrêtée avant que le groupe cyano du produit désiré (formule V) ait été transformé de façon importante par la mononitrilase.

4. Procédé selon l'une quelconque des revendications précédentes, caractérisé en ce que moins de 25 %, de préférence moins de 5 %, du produit désiré (formule V) ont été transformés.

5. Procédé selon l'une quelconque des revendications précédentes, caractérisé en ce que le dinitrile (formule IV) est prochiral et que le produit désiré (formule V) est chiral.

6. Procédé selon l'une quelconque des revendications précédentes, dans lequel la mononitrilase est une enzyme immobilisée.

7. Procédé selon l'une quelconque des revendications précédentes, caractérisé en ce que la mononitrilase manifeste des propriétés enzymatiques, chimiques et immunologiques essentiellement identiques à celles de la mononitrilase dérivée des souches déposées sous les n° NCIB 12067 à 12073.

8. Procédé selon l'une quelconque des revendications précédentes, dans lequel le dinitrile est choisi dans le groupe constitué par le dinitrile de l'acide malonique, le dinitrile de l'acide glutarique, le dinitrile de l'acide adipique, le dinitrile de l'acide sébacique, le dinitrile de l'acide 3,3-diméthylglutarique, le dinitrile de l'acide 3-méthyl-3-hydroxyglutarique, le dinitrile de l'acide 3-éthyl-3-aminoglutarique et le 1,4-dicyanobenzène.

9. Procédé de préparation de Nylon 6, qui comprend la préparation d'un composé de formule générale Va
NC-(CH₂)₄-COR¹ (Va)
dans laquelle R¹ est tel que défini dans la revendication 2, par le procédé selon l'une quelconque des revendications précédentes.

10. Procédé selon la revendication 8, dans lequel le dinitrile est le dinitrile de l'acide adipique.
